Europäisches Patentamt

European Patent Office

Office européen des brevets

(11) Publication number: **0 505 962 A1**

# EUROPEAN PATENT APPLICATION

(21) Application number: **92104990.4**

(22) Date of filing: **23.03.92**

(51) Int. Cl.⁵: **A61L 25/00**, A61K 37/12, A61J 1/00

(30) Priority: **29.03.91 IT MO910046**

(43) Date of publication of application:
**30.09.92 Bulletin 92/40**

(84) Designated Contracting States:
**AT BE CH DE DK ES FR GB GR IT LI NL SE**

(71) Applicant: **MIRAMED S.p.A.**
**Via Morandi 6**
**I-41037 Mirandola (Modena)(IT)**

(72) Inventor: **Ghedini, Claudia**
**Via Raimondi 11**
**I-40100 Bologna(IT)**

(74) Representative: **Modiano, Guido et al**
**c/o Modiano & Associati S.r.l. Via Meravigli, 16**
**I-20123 Milano(IT)**

(54) **Method and pre-assembled kit for obtaining fibrogen in a full sterile environment.**

(57) The pre-assembled kit (1) for obtaining fibrin sponge comprises a pair of bags (2,3) each having an access connection (2a,3a) closable by means of a plug (2b,3b) and being interconnected by a tube (4). One of the bags (3) has a duct for connection to a plasmapheresis unit. The inner surfaces of the walls of the bags (2,3) for containing plasma have knurlings formed thereon for retaining cryoprecipitate containing a high amount of fibrinogen. A process is also disclosed wherein the pre-assembled kit is used for obtaining fibrin sponge in a fully sterile environment.

FIG.1

The present invention relates to a method and to a pre-assembled kit for obtaining fibrin sponge (fibrin glue) in a fully sterile environment.

Fibrin sponge, constituted by two essential components, fibrinogen and thrombin, has been used for a long time in the surgical field for various kinds of suture.

The first of these components, fibrinogen, can be obtained from the plasma of a group of donors, as occurs for so-called commercial fibrinogen, or can be obtained at transfusion centers with known cryoprecipitation methods; autologous or homologous fibrinogen is obtained in this case.

In both cases, anyway, open containers are used to obtain fibrin sponge; human plasma is collected therein and said containers, together with the plasma, are subjected to repeated freezing and thawing cycles, indicatively with temperature ranges comprised between -80°C and +4°C, for approximately 20 hours, and to centrifugation, so as to separate, due to difference in density, the plasma from the fibrinogen cryoprecipitate.

The latter is recovered from the container used for centrifugation and is transferred into a container in order to have it available for use.

The above described known method has a substantial and considerable defect: the various treatments to which the open containers must be subjected during the fibrinogen preparation cycle do not at all ensure the necessary sterility of the system.

Furthermore, since said containers, usually bags, are made of PVC, this material is scarcely suitable for the repetition of freezing and thawing cycles and becomes very fragile and porous.

The aim of the present invention is to provide a method and a pre-assembled kit for obtaining fibrin sponge in a fully sterile environment which allows any treatment and process without affecting the mechanical strength of the containers and the absolute sterility of the system.

This aim and other objects are achieved by a method for obtaining fibrin sponge in a fully sterile environment, characterized in that it consists in directly drawing from a plasmapheresis unit a preset volume of plasma from a patient, introducing it, through at least one related duct provided with a coupling, into a pre-assembled kit, subsequently tight-seal closing said duct directly downstream of said coupling, then eliminating said coupling, subsequently subjecting the kit to conventional and repeated cryoprecipitation cycles by means of a series of freezing and thawing processes with the consequent separation of fibrinogen from the plasma.

Advantageously, the pre-assembled kit for carrying out the process for obtaining fibrin sponge in a fully sterile environment is characterized in that it comprises at least one pair of bags made of flexible polymeric material, which have a preset capacity and are monolithically mutually interconnected by means of at least one common tube, said bags being both provided with at least one access connection of the membrane type, which can be closed by a related plug, at least one of said bags furthermore having a duct for direct connection to a plasmapheresis unit, said bags being internally provided with wall surfaces affected by knurlings which are suitable for facilitating the cohesion retention of the cryoprecipitate.

Further characteristics and advantages of the invention will become apparent from the description of a preferred but not exclusive embodiment of a method and pre-assembled kit for obtaining fibrin sponge in a fully sterile environment, said kit being illustrated in the accompanying drawings wherein:

Figure 1 is a front view of the pre-assembled kit for obtaining fibrin sponge in a fully sterile environment;

Figure 2 is an enlarged sectional view taken along the section line II-II of figure 1, illustrating the wall of a bag for containing plasma which has knurlings formed in its inner surface for retaining cryoprecipitate containing a high amount of fibrinogen.

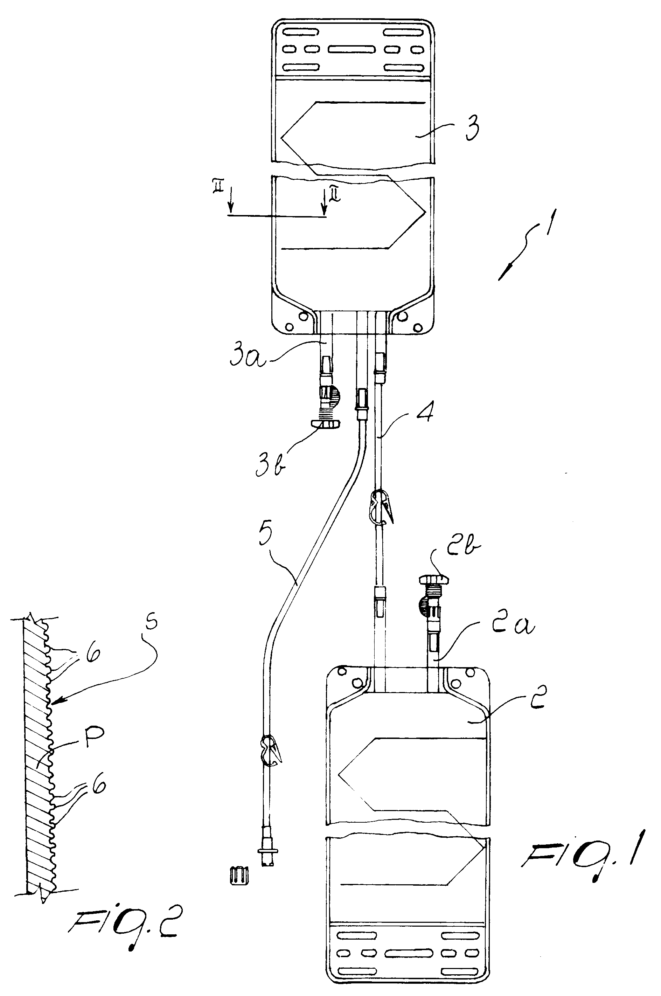

With particular reference to figure 1, the reference numeral 1 generally indicates a pre-assembled kit for obtaining fibrin sponge in a fully sterile environment, which is constituted by a pair of bags 2 and 3 made of flexible polymeric material which have a preset capacity and are monolithically mutually interconnected by means of a common tube 4.

Each one of the bags 2 and 3 is furthermore provided with an access connection, respectively indicated by 2a and 3a, which can be closed by means of a respective plug, 2b and 3b, and at least one bag, the one indicated by 3 in the figure, is furthermore provided with a duct 5 for connection to a plasmapheresis unit.

Finally, the internal surfaces "S" of the walls P of the bags 2 and 3 are affected by knurlings 6 which are suitable for facilitating the cohesion retention of the cryoprecipitate.

The process carried out for obtaining fibrin sponge in a fully sterile environment and the use of the related preassembled kit is as follows:

the two bags 2 and 3, which preferably have a capacity of 1000 cc, are mutually monolithically interconnected by means of the common tube 4, and the entire kit 1 is directly connected to a known plasmapheresis unit via the connecting duct 5.

Via this last duct, the plasma which arrives from said unit is collected in the bag 3 and said duct 5 is then closed, usually by means of a heat-welding performed proximate to the access connection 3a, and the excess portion is eliminated.

The entire kit 1 is then subjected to a sequence of cycles of freezing (down to -80°C and for 20 hours) and thawing (up to +4°C) and centrifugation (on the average around 3000 rpm and for 15 minutes) so as to obtain, still in the bag 3, a cryoprecipitate which contains a high amount of fibrinogen, substantially equal to the one present in commercial fibrin sponge.

The fluid is subsequently transferred by gravity into the bag 2, while the cryoprecipitate remains in the bag 3 by clinging to the inner surfaces S of the walls P which are affected by knurlings 6 for this purpose.

Once transfer has been completed, the common tube 4 is closed, again by heat welding, in two points, each located proximate to the bags 2 and 3; said bags are then separated one from the other by cutting said common tube between the executed heat-weldings.

The bags 2 and 3 are then stored in a conventional manner ready for use.

They are preferably manufactured by using a flexible polymeric material, preferably a copolymer made of polyethylene and vinyl acetate, the latter being present in percentages comprised between 18% and 28%.

In practice it has been observed that the invention thus described achieves the intended aim and objects, i.e. it allows to obtain fibrin sponge without contact with the external environment during preparation, and thus with an absolute assurance of asepsis.

The invention thus conceived is susceptible to modifications and variations, all of which are within the scope of the inventive concept.

All the details may furthermore be replaced with other technically equivalent elements.

In practice, the materials employed, as well as the dimensions, may be any according to the requirements without thereby abandoning the scope of the protection of the following claims.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Method for obtaining fibrin sponge in a fully sterile environment, characterized in that it consists in directly drawing from a plasmapheresis unit a preset volume of plasma from a patient, introducing it, through at least one related duct (5) provided with a coupling, into a pre-assembled kit (1), subsequently tight-seal closing said duct (5) directly after said coupling, then in eliminating the latter, subsequently subjecting the kit (1) to conventional and repeated cryoprecipitation cycles by means of a series of freezing and thawing processes with consequent separation of fibrinogen from the plasma.

2. Pre-assembled kit for obtaining fibrin sponge in a fully sterile environment, characterized in that it comprises at least one pair of bags (2,3) made of flexible polymeric material which have a preset capacity and are mutually monolithically interconnected by means of at least one common tube (4), both of said bags (2,3) being provided with at least one access connection (2a,3a) of the membrane type which can be closed by a related plug (2b,3b), at least one of said bags (3) being furthermore provided with a duct (5) for direct connection to a plasmapheresis unit, said bags (2,3) having internal walls (P) provided with surfaces (S) which are affected by knurlings (6) suitable for facilitating the cohesion retention of the cryoprecipitate.

3. Method according to claim 1, characterized in that once the cryoprecipitation cycles have been completed, the residual fluid plasma is separated from the cryoprecipitate, the latter being kept in a first one of said bags (3), and is introduced into the second bag (2), said bags (2,3) being furthermore sealed and separated one from the other after closing said common tube (4) by heat sealing.

4. Pre-assembled kit for obtaining fibrin sponge in a fully sterile environment according to claim 2, characterized in that said flexible polymeric material is made of a copolymer which is composed of polyethylene and vinyl acetate.

5. Pre-assembled kit for obtaining fibrin sponge in a fully sterile environment according to claim 4, characterized in that the vinyl acetate is present in said copolymer in percentages comprised between 14% and 30%.

Fig.1

Fig.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X<br>A | FR-A-2 283 700 (BAXTER)<br><br>* page 2, line 6 - page 3, line 1; figure 1 * | 1<br>2 | A61L25/00<br>A61K37/12<br>A61J1/00 |
| A | MANUILA 'dictionnaire français de medicine et de biologie  vol 11'<br>1971 , MASSON , PARIS<br>* page 201, column 2, line 19 - line 22 * | 1 | |
| PA | WO-A-9 109 573 (B MORSE)<br>* page 8, line 10 - page 13, line 22; figure 1 * | 1,2,3 | |
| A | GB-A-2 001 657 (BAXTER TRAVENOL)<br>* the whole document * | 4,5 | |
| A | FR-A-2 439 589 (BAXTER TRAVENOL)<br>* page 1, line 1 - line 9 *<br>* page 6, line 21 - page 7, line 6; figure 1 * | 2-5 | |

TECHNICAL FIELDS
SEARCHED (Int. Cl.5 )

A61L
A61K
A61J

The present search report has been drawn up for all claims

| Place of search | Date of completion of the search | Examiner |
|---|---|---|
| THE HAGUE | 25 JUNE 1992 | VEREECKE A. |